# EUROPÄISCHE PATENTSCHRIFT

(11) **EP 2 212 214 B1**
(45) Veröffentlichungstag und Bekanntmachung des Hinweises auf die Patenterteilung: **10.08.2011**
(21) Anmeldenummer: 08854522.3
(22) Anmeldetag: 31.10.2008
(51) Int. Cl.: B65D 51/20

(54) **FLACHER BEHÄLTER**
FLAT CONTAINER
RÉCIPIENT PLAT

(30) Priorität: 26.11.2007 CH 18212007; 17.10.2008 CH 16502008
(43) Veröffentlichungstag der Anmeldung: 04.08.2010
(73) Patentinhaber: Hoffmann Neopac AG, 3602 Thun (CH)
(72) Erfinder: WICHOWSKI, Artur, CH-4563 Gerlafingen (CH)
(74) Vertreter: Gachnang, Hans Rudolf
(86) Internationale Anmeldenummer: PCT/CH2008/000457
(87) Internationale Veröffentlichungsnummer: WO 2009/067827

(56) Entgegenhaltungen:
- BE-A- 390 753
- US-A- 2 044 677
- US-A1- 2005 109 781
- US-B1- 7 124 883

## Beschreibung

Gegenstand der Erfindung ist ein flacher Behälter für Zigaretten, Zigarren, Süssigkeiten und dergleichen gemäss Oberbegriff des Patentanspruchs 1.

Teure Zigaretten, Zigarren, Zigarillos und dergleichen aber auch Süssigkeiten werden oft in Blechbehältern verkauft, da sie darin einerseits gegen Beschädigung geschützt aufbewahrt werden können und zudem bis zur Erstöffnung eine grosse Luft- und Wasserdampfdichtigkeit gewährleistet ist. Letzteres ist ein Problem, denn das Verschliessen von Blechbehältern mit angelenktem Deckel ist schwierig, weshalb oft die Ware beispielsweise in Aluminiumfolie oder ein Laminat eingeschweisst und danach erst in die Dose eingelegt wird.

Aus der US 7,124,883 B1 ist ein Zigarrenbehälter Gemäß dem Oberbegriff des Anspruchs 1 bekannt mit einem aus Kunststoff bestehenden zentralen Teil, der einen Zwischenboden aufweist und den Behälter in einen Oberteil mit einem ersten Deckel und einen Unterteil mit einem zweiten Deckel teilt. Auf die breiten Kanten des Zwischenteils sind Folien aufgeklebt, um die Behälterteile luftdicht zu verschliessen. Zudem sind der obere und der untere Deckel mittels aufgeklebten Scharnieren mit dem zentralen Teil verbunden. Die Herstellung eines solchen Behälters ist aufwendig und das Aufsiegeln einer Verschlussmembran muss in zwei Schritten, nämlich nach dem Füllen des oberen Behälterteils mit Zigaretten oder Zigarren und nach dem Füllen des unteren Teils folgen. Zudem müssen die beiden Deckel zuerst mit einem Scharnierband versehen werden und letzteres nach dem Verkleben der Verschlussmembranen an den Behälterrand angebracht werden.

Aus der US 2005/0109781 A1 ist ein Behälter für Lebensmittel oder Getränke bekannt, der die Gestalt einer Dose aufweist und durch eine Folie versiegelbar ist. Bei diesem bekannten Behälter ist der Deckel nicht Teil der Dose und er ist auf einem Wulst am oberen Rand des Behälters aufgesteckt.

Eine Aufgabe der vorliegenden Erfindung besteht nun darin, den Unterteil des Behälters, d.h. Behälterunterteil, in welchem die Ware (Zigaretten, Zigarren etc.) liegen, so luft- und wasserdampfdicht wie möglich abzuschliessen. Eine weitere Aufgabe der Erfindung besteht darin, dass der die Luft- und Wasserdampfdichtigkeit bewirkende Verschluss werkzeugfrei vom Behälterunterteil abnehmbar ist.

Gelöst wird diese Aufgabe durch einen Behälter mit den Merkmalen des Patentanspruchs 1.

Vorteilhafte Ausgestaltungen des Behälters sind in den abhängigen Ansprüchen umschrieben.

Durch eine geeignete Formgebung des Behälterunterteils kann in diesem eine Folie oder Membran eingeklebt oder eingesiegelt werden. Die Siegelfläche wird dabei durch einen Absatz mit einer Schulter, die eine genügend grosse Klebefläche zwischen Membran und Behälterunterteil.ergibt, gebildet. Das Einsiegeln der Membran nach der Befüllung des Behälterunterteils mit der zu stauenden Ware kann auf einfachste Art und Weise durch einen beheizten Stempel erfolgen. Durch die vollflächige Auflage des Bechälterunterteils auf einer Abstützfläche kann die Siegelwärme optimal abgeleitet und damit das Füllgut vor Wärme geschützt werden. Auch findet dabei keine Verformung des Behälterunterteils statt. Die erfindungsgemässe Ausformung der Siegelfläche erlaubt eine präzise Zentrierung der Siegelmembran vollständig im Innern der Dose. Das Ablösen/Abschälen der Membran ist ohne Zuhilfenahme von Werkzeugen leicht von Hand möglich. Dabei wird die gesamte Behälterquerschnittsfläche rückstandsfrei freigelegt. Der Behälter ist zudem kostengünstig durch einen Tiefziehvorgang herstellbar und nach Entleerung vollständig rezyklierbar, da keine Kunststoffteile mit dem Behälter verbunden sind.

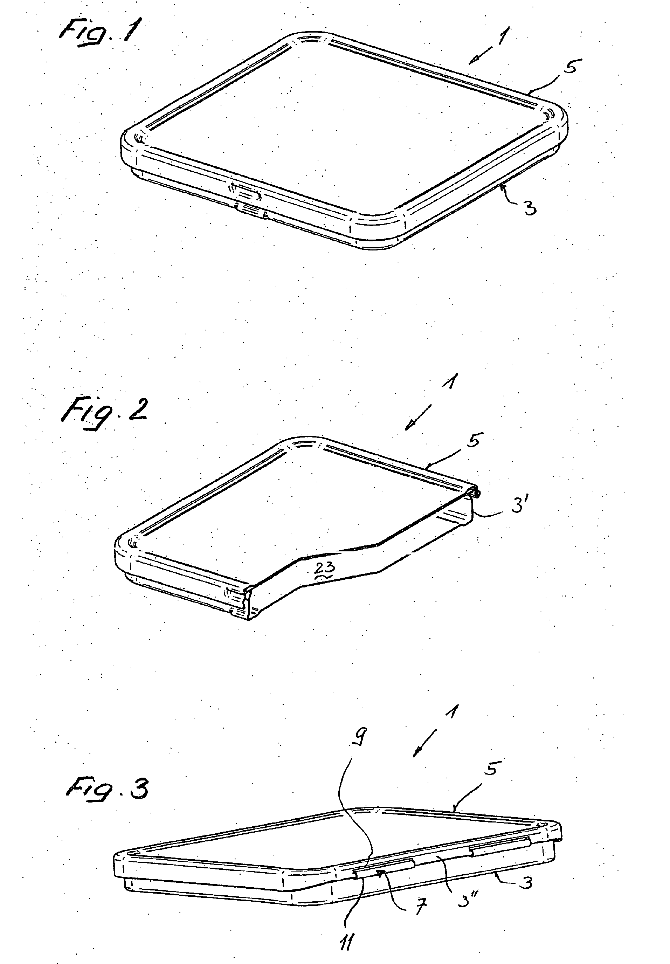

Anhand eines illustrierten Ausführungsbeispieles wird die Erfindung näher erläutert. Es zeigen
- Figur: 1 eine perspektivische Darstellung eines geschlossenen Behälters mit einem Behälterunterteil und einem Behälteroberteil, .'
- Figur 2: eine perspektivische Darstellung des Behälters gemäss Figur 1, vordere Hälfte aufgeschnitten,
- Figur 3: eine perspektivische Darstellung des Behälters von hinten betrachtet,

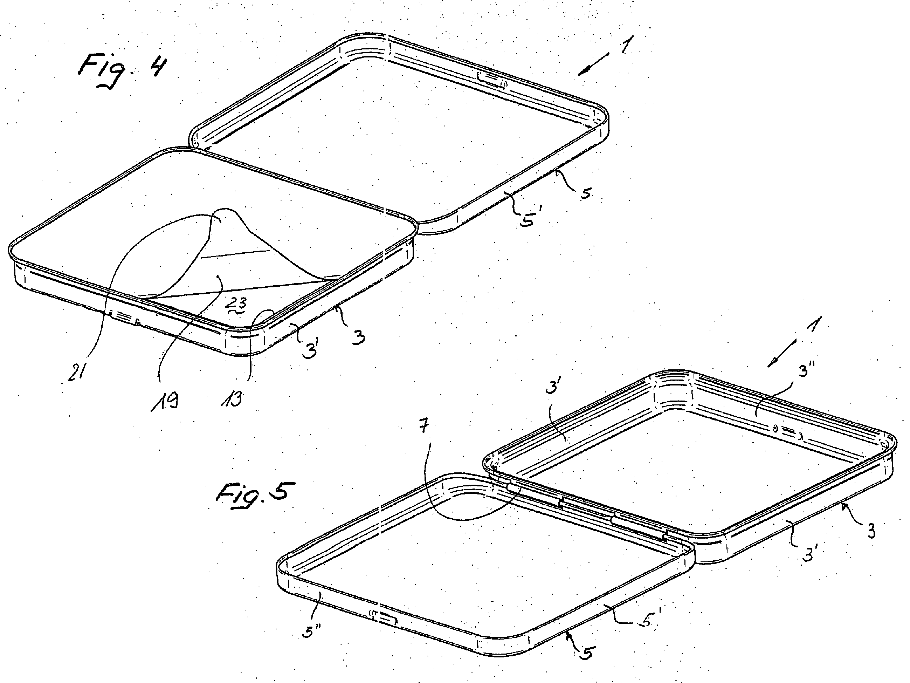

- Figur 4: eine perspektivische Darstellung des geöffneten Behälters mit teilweise abgelöster Verschlussmembran,
- Figur 5: eine perspektivische Darstellung des geöffneten Behälters, Membran abgenommen,
- Figur 6: einen vergrösserten Querschnitt durch den hinteren Randbereich des Behälters ohne eingesiegelte Folie,
- Figur 7: einen vergrösserten Querschnitt durch den hinteren Randbereich des Behälters mit eingesiegelter Folie,
- Figur 8a: einen Teilquerschnitt durch eine weitere Ausgestaltung des Behälterunterteils mit aufgesetztem Behälteroberteil,
- Figur 8b: einen Teilquerschnitt durch den Behälterunterteil in Figur 8a ohne Deckel,
- Figur 8c: einen Teilquerschnitt durch den Behälter mit eingefahrener Siegelfläche und
- Figur 8d: einen Teilquerschnitt durch den Behälter mit eingefahrener Siegelfläche und von oben wirkendem Heizwerkzeug.

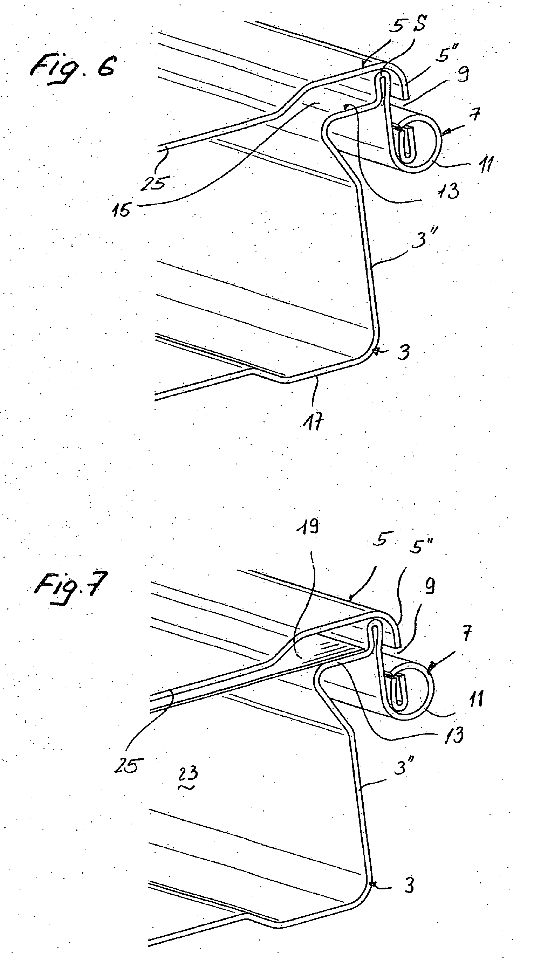

Mit Bezugszeichen 1 ist in den Figuren ein Behälter bezeichnet, welcher einen Behälterunterteil 3 und einen Behälteroberteil 5 umfasst. Sowohl der Behälterunterteil 3 als auch der Behälteroberteil 5 weisen umlaufende Seitenwände 3' und 3" bzw. 5' und 5" auf. Die beiden Behälterteile 3,5 sind durch Scharniere 7 miteinander verbunden. Die Scharniere 7 werden durch geeignet geformte Lappen an den Kanten der Behälterunter- und -oberteile gebildet. Am Behälteroberteil 5 sind in dessen einen Seitenwand 5" im vorliegenden Beispiel zwei rechteckförmige Ausnehmungen 9, welche parallel zu den Kanten verlaufen, ausgestanzt. In die beiden Ausnehmungen 9 greifen Laschen 11 ein, welche Teil der Seitenwand 3" sind, und durch einen Umformprozess (Umbiegen/Einrollen) erzeugt worden sind. Der Behälterunterteil 3 ist folglich nicht von Ausstanzungen und dergleichen durchbrochen. Im oberen Bereich der Seitenwände 3' und 3" des Behälterunterteils 3 ist durch einen Verforrmungsvorgang ein nasenförmiger nach innen gerichteter Absatz 13 ausgebildet. Am Absatz 13 verläuft die obenliegende Fläche 15 parallel zum Boden 17 und der freien Oberkante (Scheitel S) des Behälterunterteils 3. Die Fläche 15 erstreckt sich entlang dem gesamten Umfang des Behälterunterteils 3 (vergleiche insbesondere Figuren 6 und 7). Die Fläche 15 liegt in einem vertikalen Abstand vom Scheitel S der Seitenwände 3' und 3", d.h. die Fläche ist beabstandet von der Oberkante. Sie bildet damit eine umlaufende streifenförmige plane Auflage für eine Membran oder Folie 19 (Figur 7). Letztere wird durch einen Kleber auf dem Absatz 3 schälbar gehalten. Als Kleber kann ein Hotmelt verwendet oder eingesetzt sein oder die innere Oberfläche des unteren Behälterteils 3 ist mit einem siegelbaren Lack (ein PE-, PVC- oder PP-haltiger Lack) beschichtet, ebenso die Unterseite der Folie 19. Um die Folie 19 leicht vom Absatz 13 abschälen zu können, muss der Kleber einerseits schälbar sein und an der Folie 19 ist eine Lasche 21 angeformt, welche nach dem Aufsiegeln der Folie 19 auf dem Absatz 13 leicht mit zwei Fingern ergreifbar nach oben absteht.

Das Aufbringen der Siegelfolie 19 auf dem Absatz 13 nach dem Einfüllen des Füllguts (Zigaretten, Zigarillos und dergleichen) kann auf einfachste Weise mit einem der Kontur des Absatzes 13 folgenden beheizten Siegelwerkzeugs oder durch eine Ultraschallschweissung erfolgen. Die Abstützung des Siegeldruckes kann auf einfache Weise von aussen erfolgen. Zudem ist die durch die Siegelung entstehende Wärme weit beabstandet vom Füllgut. Da im unteren Behälterteil 3 keine Ausstanzungen, Ausnehmungen und dergleichen angebracht sind, ist der unter der Folie 19 liegende Füllgutraum 23 nach dem Aufsiegeln luft- und wasserdampfdicht verschlossen und gewährleistet optimale Verhältnisse für das Füllgut.

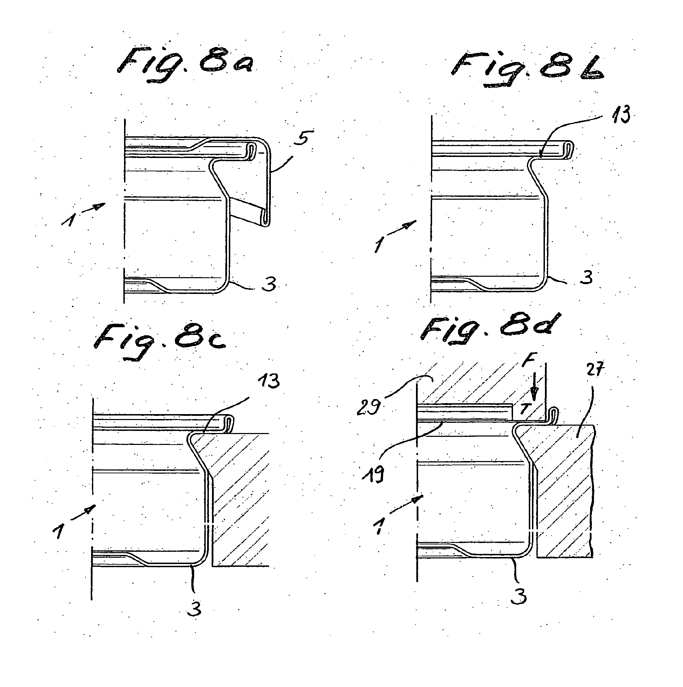

In der Ausgestaltung des Behälters 1 gemäss den Figuren 8a bis 8d ist am Behälterunterteil 3 wiederum ein breiter Absatz als Siegelfläche ausgebildet. Die Verbindung zwischen Behälterunterteil 3 und Deckel 5 ist hier nicht näher dargestellt; sie kann wiederum durch ein Scharnier gemäss den Figuren 6 und 7 oder auf andere Weise erfolgen.

Um eine einwandfreie Siegelverbindung zwischen der Folie 19 und dem Absatz 13 (der Siegelfläche) zu erlangen, wird beim Siegeln eine den Absatz 13 untergreifende Stützvorrichtung 27 unter den Absatz 13 geführt. Vorzugsweise ist die Stützvorrichtung 27 derart ausgebildet, dass sie mindestens unterhalb des Absatzes 13 vollflächig an diesem anliegt. Mit einem Siegelwerkzeug 29, welches sich wiederum über die gesamte Breite des Absatzes 13 erstreckt, kann eine optimale vollflächige Anlage zwischen Absatz 13, Folie 19 und dem Siegelwerkzeug 29 erlangt werden, ohne dass der Behälterunterteil 3 axiale Kräfte F aufnehmen muss.

Im weiteren wird die vom Siegelwerkzeug 29 erzeugte Temperatur T im Siegelbereich direkt durch die Stützvorrichtung 27 abgeleitet. Eine Beeinträchtigung des Füllgutes durch eine Temperaturerhöhung im Randbereich des Behälters 1 wird dadurch vermieden.

Am Behälteroberteil 5 ist Vorzugsweise die zwischen den Rändern 5' und 5" liegende Fläche durch einen Tiefziehvorgang abgetieft, d.h. der zentrale Bereich des Behälteroberteils liegt tiefer als dessen Rand. Aus Figur 6 ist leicht ersichtlich, dass der zentrale Teil 25 parallel zur Folie 19 verläuft und in sehr geringem Abstand oder ohne Abstand zu dieser liegt. Durch diese Massnahme ist die meist sehr dünne Folie 19 gegen Beschädigung durch dass Füllgut im Füllraum 23 geschützt, wenn der Behälter 1 z.B. fallengelassen wird. Anders gesagt, es kann eine wesentlich dünnere Folie 19 eingesetzt werden, da diese keinen mechanischen Beanspruchungen ausgesetzt wird.

## Patentansprüche

1. Flacher Behälter (1) aus einem durch Tiefziehen umformbaren Material für Zigaretten, Zigarren, Süssigkeiten und dergleichen mit einem Behälterunterteil (3) und einem Behälteroberteil (5), welche beiden Teile (3,5) durch mindestens ein durch einen Umformprozess erzeugtes Scharnier (7) gelenkig miteinander verbunden sind,
**dadurch gekennzeichnet,**
**dass** am Behälterunterteil (3) ein umlaufender Absatz (13) als Siegelfläche (15) ausgebildet ist, welcher zum Aufsiegeln einer den Füllraum (23) im Behälterunterteil (3) oben luftdicht abschliessenden Folie (19) ausgebildet ist, und dass der umlaufende Absatz (13) durch eine umlaufende, nach innen gerichtete Sicke am Behälterunterteil (3) gebildet wird, und dass die Siegelfläche (15) des Absatzes (13) in einem Abstand zur Behälteroberkante (Scheitel S) angeordnet ist, und dass die Scharniere (7) an den Kanten des Behälterunterteils (3) ausserhalb einer Bahälterwand (3") des Behälters (3) ausgebildet sind.

2. Behälter nach Anspruch 1, **dadurch gekennzeichnet, dass** auf der Siegelfläche (15) des Absatzes (13) ein Kleber aufgebracht ist oder dass die Innenseite des unteren Behälterteils (3) und die Unterseite der Folie (19) mit einem siegelbaren Lack beschichtet sind.

3. Behälter nach einem der Ansprüche 1 oder 2, **dadurch gekennzeichnet, dass** der die Siegelfläche (15) bildende Absatz (13) im wesentlichen vollständig innerhalb einer Seitenwand (3") des Behälters (1) liegt.

4. Behälter nach Anspruch 3, **dadurch gekennzeichnet, dass** der Absatz (13) durch einen Verformungsvorgang nasenförmig und nach innen gerichtet ausgebildet ist.

5. Behälter nach Anspruch 4, **dadurch gekennzeichnet, dass** die oben liegende Fläche (15) des Absatzes (13) parallel zum Boden (17) liegt, derart dass eine Abstützung des Siegeldruckes von aussen erfolgen kann.

6. Behälter nach einem der Ansprüche 3 bis 5, **dadurch gekennzeichnet, dass** ausserhalb der Siegelfläche (15) ein umlaufender Rand angebracht ist und zur Auflage des Deckelteils (5) und zur Zentrierung der Folie (19) ausgebildet ist.

7. Flacher Behälter nach einem der Ansprüche 1 bis 6, **dadurch gekennzeichnet, dass** der zentrale Teil (25) des oberen Behälterteils (5) abgesenkt ist.

8. Flacher Behälter nach Anspruch 7, **dadurch gekennzeichnet, dass** der zentrale Teil (25) des oberen Behälterteils (5) bei geschlossenem Behälter (1) in geringem Abstand zur Folie liegt oder an dieser anliegt.

## Claims

1. A flat container (1) from a material which may be formed by way of deep-drawing, for cigarettes, cigars, sweets and likewise, with a container lower part (3) and with a container upper part (5), said two parts (3, 5) being connected to one another in an articulated manner by way of at least one hinge (7) which is produced by a forming process,
**characterised in that**
a peripheral shoulder (13) as a sealing surface (15) is formed on the container lower part (3) and is designed for the sealing-on of a film (19) which closes the filling space (23) in the container lower part (3) to the top in an airtight manner, and that the peripheral shoulder (13) is formed by a peripheral, inwardly directed bead on the container lower part (3), and that the sealing surface (15) of the shoulder (13) is arranged at a distance to the container upper edge (apex S), and that the hinges (7) are formed on the edges of the container lower part (3) outside a container wall (3") of the container (3).

2. A container according to claim 1, **characterised in that** an adhesive is deposited on the sealing surface (15) of the shoulder (13) or that the inner side of the lower container part (3) and the lower side of the film (19) are coated with a sealable coating.

3. A container according to one of the claims 1 or 2, **characterised in that** the shoulder (13) forming the sealing surface (15) lies essentially completely within a side wall (3") of the container (1).

4. A container according to claim 3, **characterised in that** the shoulder (13), by way of a shaping procedure, is lug-like and is designed in an inwardly directed manner.

5. A container according to claim 4, **characterised in that** the upper lying surface (15) of the shoulder (13) lies parallel to the base (17) in a manner such that a support of the sealing pressure from the outside may be effected.

6. A container according to one of the claims 3 to 5, **characterised in that** a peripheral edge is incorporated outside the sealing surface (15) and is designed for the resting of the cover part (5) and for centering the film (19).

7. A flat container according to one of the claims 1 to 6, **characterised in that** the central part (25) of the upper container part (5) is sunk.

8. A flat container according to claim 7, **characterised in that** the central part (25) of the upper container part (25), given a closed container (1), lies at a small distance to the film or bears on this.

## Revendications

1. Récipient plat (1) constitué d'un matériau qui peut être conformé par emboutissage en profondeur pour des cigarettes, des cigares, des sucreries et similaires, comprenant une partie inférieure de récipient (3) et une partie supérieure de récipient (5), dont ces deux parties (3, 5) sont reliées entre elles de manière articulée par au moins une charnière (7) générée par un processus de façonnage,
**caractérisé en ce que**
sur la partie inférieure de récipient (3), un gradin périphérique (13) est réalisé comme surface de scellement (15), laquelle est réalisée pour le scellement d'un film (19) qui ferme le compartiment de remplissage (23) par le haut et de manière étanche à l'air dans la partie inférieure de récipient (3) et **en ce que** le gradin périphérique (13) est formé par une moulure périphérique et dirigée vers l'intérieur sur la partie inférieure de récipient (3) et **en ce que** la surface de scellement (15) du gradin (13) est agencée à une distance par rapport à l'arête supérieure du récipient (sommet S) et **en ce que** les charnières (7) sont réalisées sur les arêtes de la partie inférieure de récipient (1) en dehors d'une paroi de récipient (3") du récipient (1).

2. Récipient selon la revendication 1, **caractérisé en ce que**, sur la surface de scellement (15) du gradin (13), une colle est apposée ou **en ce que** la face intérieure de la partie inférieure de récipient (3) et la face de dessous du film (19) sont enduites d'un vernis qui peut être scellé.

3. Récipient selon l'une ou l'autre des revendications 1 et 2, **caractérisé en ce que** le gradin (13) qui forme la surface de scellement (15) se trouve essentiellement au complet à l'intérieur d'une paroi latérale (3") du récipient (1).

4. Récipient selon la revendication 3, **caractérisé en ce que** le gradin (13) est réalisé par une procédure de déformation en forme de nez et est réalisé en étant orienté vers l'intérieur.

5. Récipient selon la revendication 4, **caractérisé en ce que** la surface placée en haut (15) du gradin (13) est placée parallèlement au fond (17) de telle sorte qu'un soutien de la pression de scellement peut avoir lieu depuis l'extérieur.

6. Récipient selon l'une des revendications 3 à 5, **caractérisé en ce que**, en dehors de la surface de scellement (15), une bordure périphérique est ménagée et est réalisée pour l'appui de la partie de couvercle (5) et pour le centrage du film (19).

7. Récipient plat selon l'une des revendications 1 à 6, **caractérisé en ce que** la partie centrale (25) de la partie supérieure de récipient (5) est abaissée.

8. Récipient plat selon la revendication 7, **caractérisé en ce que** la partie centrale (25) de la partie supérieure de récipient (5) se trouve à une faible distance par rapport au film (19) lorsque le récipient (1) est fermé.
